# FASCICULE DE BREVET EUROPEEN

(11) **EP 2 858 580 B1**
(45) Date de publication et mention de la délivrance du brevet: **04.05.2016**
(21) Numéro de dépôt: 13727916.2
(22) Date de dépôt: 11.06.2013
(51) Int. Cl.: A61B 17/064, A61B 17/70

(54) **SYSTÈME DE FIXATION POUR FACETTES ARTICULAIRES ET SES APPLICATIONS**
BEFESTIGUNGSSYSTEM FÜR GELENKIGE FACETTEN UND ANWENDUNGEN DAVON
FIXING SYSTEM FOR ARTICULAR FACETS AND APPLICATIONS THEREOF

(30) Priorité: 12.06.2012 FR 1255507
(43) Date de publication de la demande: 15.04.2015
(73) Titulaire: Spineart SA, 1217 Meyrin (CH)
(72) Inventeur: LEVIEUX, Jérôme, Laguna Beach, California 92651 (US)
(74) Mandataire: Cabinet Plasseraud
(86) Numéro de dépôt international: PCT/EP2013/062014
(87) Numéro de publication internationale: WO 2013/186205

(56) Documents cités:
- GB-A- 2 471 648
- US-A1- 2010 082 065
- US-A1- 2010 145 391
- US-A1- 2010 280 555

## Description

La présente invention concerne un système de fixation pour facettes articulaires et ses applications.

En chirurgie du rachis, il est parfois nécessaire de bloquer en position les facettes articulaires postérieures (parfois appelées "articulaires postérieures") d'une articulation entre deux vertèbres. Ce blocage est destiné à favoriser l'arthrodèse de l'articulation vertébrale dans le but par exemple de soulager des douleurs. Les facettes articulaires permettent la flexion et la rotation des vertèbres l'une par rapport à l'autre et leurs surfaces articulaires sont recouvertes de cartilage lisse.

Les techniques mini-invasives se développant fortement, les systèmes devraient permettre à un chirurgien de réaliser l'opération par de petites incisions et en épargnant le plus possible les masses musculaires.

Une technique connue de fixation des facettes articulaires est la mise en place d'une vis transfixante. Cette technique présente l'avantage d'être réalisable avec des vis classiques, peu onéreuses, et d'autoriser l'utilisation des techniques mini-invasives. Un inconvénient important réside dans la difficulté d'implantation, car la visée doit être très précise afin d'éviter que la vis ne touche une racine nerveuse ou un vaisseau sanguin.

Une autre technique consiste en une pince articulée qui enserre une pièce située dans l'espace inter-articulaire comme décrit dans WO2009006622. Cette pince comprend une paire d'éléments de serrage articulés et une vis d'ancrage facultative qui coopèrent pour fixer solidement le dispositif de fusion à une articulation à facette de la colonne vertébrale. On fait pivoter ensemble les éléments de serrage pour pincer les facettes osseuses adjacentes et ainsi promouvoir leur fusion. A cette fin, un écrou à embase circulaire est vissé sur une vis bissectrice installée entre les éléments de serrage pour rapprocher ces éléments de serrage et ainsi pincer les facettes osseuses.

Ce système présente l'avantage d'éviter l'utilisation de vis transfixante, et donc des inconvénients associés. De plus, la vis bissectrice favorise la fusion car les facettes articulaires ne sont pas au contact direct l'une de l'autre sur une surface cartilagineuse plus propice au glissement qu'à la fusion.

Mais ce système présente plusieurs inconvénients majeurs:
- la mise en place n'est pas très facile car la pince doit être écartée, descendue, puis un instrument comme une clé Allen doit serrer l'écrou qui refermera la pince.
- l'écrou de serrage peut éventuellement se desserrer dans le temps
- si la forme des facettes articulaires n'est pas propice, la pince peut éventuellement glisser et donc ne pas fonctionner.
- les coûts de fabrication de ce type d'implant sont élevés au vu de la complexité du mécanisme comportant plusieurs pièces.

US 2010/0280555 décrit une agrafe pour facettes articulaires, constituée d'un pont plat auquel sont fixées deux broches, le fond plat étant muni d'une ouverture dans laquelle est installée une vis. Dans ce cas encore, un instrument de vissage est nécessaire et le mécanisme comporte plusieurs pièces ainsi qu'un instrument de vissage.

Il serait donc souhaitable de disposer de systèmes de fixation pour facettes articulaires qui puisse s'installer en un seul geste. Il serait aussi souhaitable de disposer de tels systèmes de fixation qui soient simples, comportant peu de pièces, voire une seule.

Pour sa part, US 2010/0082065 décrit une agrafe pour facettes articulaires, pouvant être monobloc, constituée d'un pont plat muni de deux jambes, l'agrafe ayant une forme générale en U, une colonnette cylindrique terminée par un cône pointu étant installé entre les jambes.

Or après de longues recherches la demanderesse a créé un système de fixation pour facettes articulaires donnant satisfaction. Ce système est fondé sur le principe de l'agrafe, petit objet métallique servant à attacher deux ou plusieurs choses ensemble ou à réunir deux parties d'une même chose. Alors qu'une telle agrafe de type le plus classique a une forme générale de U et est habituellement constituée de deux jambes parallèles reliées par un pont perpendiculaire à ces deux jambes, l'agrafe de l'invention comprend trois jambes alignées. Par ailleurs, alors que dans les agrafes de type le plus classique les jambes et le pont sont filaires, ici ces éléments sont des lames aplaties.

Dans la présente demande et dans ce qui suit, classiquement le terme «comprend» signifie "comporte au moins". Ainsi, l'agrafe de l'invention comporte trois jambes alignées mais peut comporter des jambes supplémentaires.

C'est pourquoi la présente demande a pour objet une agrafe comprenant trois jambes alignées solidaires d'un pont, dont une jambe médiane et des jambes externes, les jambes ayant chacune deux faces et une extrémité libre, les jambes externes ayant leur extrémité libre pointue, les jambes externes ayant une face interne et une face externe, la face interne de chacune des jambes externes et les deux faces de la jambe médiane constituant des surfaces actives, les surfaces actives étant parallèles, et où la jambe médiane et le pont sont perforés, les perforations étant en communication.

Lorsque des jambes comportent une série d'aspérités anti-retour, on considère que les sommets des d'aspérités anti-retour constituent les surfaces actives.

Dans des conditions préférentielles de mise en oeuvre de l'invention, les jambes externes comportent sur leur face interne une série d'aspérités anti-retour.

Dans d'autres conditions préférentielles de mise en oeuvre de l'invention ci-dessus, la jambe médiane comporte sur chacune de ses deux faces une série d'aspérités anti-retour.

Les aspérités anti-retour sont de préférence des séries d'encoches en dents de scie.

Dans la présente demande et dans ce qui suit, lorsque l'on dit que les surfaces actives sont parallèles, il convient d'entendre que les surfaces actives sont environ parallèles, c'est-à-dire généralement parallèles, comme on le verra ci-après sur les figures.

Dans encore d'autres conditions préférentielles de mise en oeuvre de l'invention, l'angle formé par la surface active d'une jambe externe et par la face active adjacente de la jambe médiane va en s'évasant depuis le pont vers les extrémités libres. En d'autres termes, le sommet de l'angle formé par ces surfaces actives est situé du côté du pont.

Dans toujours d'autres conditions préférentielles de mise en oeuvre de l'invention, vue en élévation, la jambe médiane a une forme environ rectangulaire.

Dans encore d'autres conditions préférentielles de mise en oeuvre de l'invention, vu de dessus, le pont a une forme environ rectangulaire. Il est avantageusement arqué dans la direction de sa longueur comme montré sur les figures.

Dans toujours d'autres conditions préférentielles de mise en oeuvre de l'invention, les jambes externes aussi, vues en élévation, ont une forme environ rectangulaire. Les jambes externes peuvent toutefois avoir des formes assez diverses. En section transversale à leur longueur, elles peuvent notamment avoir une forme environ triangulaire ou semi-circulaire.

Chacune des deux jambes externes peut être réalisée en deux éléments prenant ainsi, en vue latérale dans la direction de la longueur de l'agrafe, une forme de U, ou peut être réalisée en plus de deux éléments. Une telle configuration procure une très bonne tenue de l'agrafe à largeur égale de jambe, tout en facilitant sa pénétration dans la matière osseuse. De plus on réalise une certaine économie de matière.

Comme les jambes externes, la jambe médiane aussi peut être en deux ou plus de deux éléments, ce qui permet d'introduire de la greffe entre les éléments (par exemple deux éléments) de la jambe médiane. Cette introduction peut se faire avant l'implantation de l'agrafe ou après, grâce à une lumière prévue dans le pont.

Dans des modes de réalisation particulièrement préférés, les jambes externes et la jambe médiane sont réalisées en deux éléments.

La jambe médiane peut être plate, c'est-à-dire en forme de plaque, ou en forme de coin comme représenté sur les figures. Elle peut être trapézoïdale, c'est-à-dire que sa largeur peut être différente du côté du pont et du côté de son extrémité et sera avantageusement plus large du côté du pont que du côté de son extrémité. Elle peut aussi être cylindrique, conique ou de façon générale allongée de façon à s'insérer facilement dans l'espace inter articulaire.

Les jambes, externes ou médiane, ont de préférence une épaisseur plus importante du côté du pont.

Selon la revendication 1, la jambe médiane et le pont sont perforés, dans ce cas avantageusement en communication. Il est ainsi notamment possible de greffer de la moelle osseuse en vue d'accélérer l'arthrodèse. La perforation aura une surface pouvant par exemple représenter de 5 à 50 % de la surface du pont, notamment de huit à 40 %, avantageusement de 10 à 35 %, particulièrement de 10 à 25 %. On a ainsi une bonne capacité de réception de moelle osseuse et en outre une bonne solidité de l'agrafe.

Dans encore d'autres conditions préférentielles de mise en oeuvre de l'invention, les jambes externes sont réalisées en deux éléments, et jambe médiane est réalisée en deux éléments ou est perforée et le pont est également perforé.

La longueur du pont, qui est aussi la plus grande dimension de l'agrafe, sera par exemple de 5 à 25, de préférence de 6 à 20, notamment de 7 à 15, tout particulièrement de 8 à 12 mm.

La largeur du pont sera par exemple de 1 à 15, de préférence de 2 à 12, notamment de 3 à 10, tout particulièrement de 4 à 8 mm.

Réalisé en titane (par exemple), l'épaisseur typique (hors jambe médiane) du pont sera par exemple de 0,5 à 5, de préférence de 0,7 à 4, notamment de 0,9 à 3, tout particulièrement de 1 à 2 mm.

La longueur de la jambe médiane sera par exemple de 3 à 20, de préférence de 4 à 17, notamment de 5 à 15, tout particulièrement de 6 à 12 mm.

La largeur de la jambe médiane sera par exemple de 2 à 18, de préférence de 3 à 15, notamment de 4 à 12, tout particulièrement de 5 à 10 mm.

Comme on l'a vu précédemment, cette jambe médiane peut être notamment trapézoïdale et donc sa largeur peut diminuer au fur et à mesure qu'on s'éloigne du pont.

Dans des conditions préférentielles de mise en oeuvre de l'invention, la longueur du pont est de 7 à 15 mm et la largeur du pont est de 3 à 10 mm. Dans ce cas, de préférence la longueur de la jambe médiane sera de 5 à 15 mm et la largeur de la jambe médiane sera de 4 à 12 mm ou de 3 à 12 mm.

Lorsque l'agrafe est réalisée en titane ou inox, l'épaisseur de la jambe médiane sera par exemple de 0,5 à 6, de préférence de 0,6 à 5, notamment de 0,7 à 3, tout particulièrement de 1 à 2 mm au niveau du pont pour s'amenuiser à son extrémité libre respectivement à par exemple de 0,1 à 4, de préférence de à 0,3 à 3, notamment de 0,5 à 2, tout particulièrement de 0,7 à 1 mm.

La longueur d'une jambe externe sera par exemple de 3 à 20, de préférence de 4 à 15, notamment de 5 à 12, tout particulièrement de 6 à 10 mm. Elle sera habituellement plus courte que la jambe médiane. Ainsi, on peut commencer à insérer la jambe médiane avant de planter les jambes externes, avec pour conséquence l'assurance d'un bon centrage. Sa longueur sera avantageusement de 0,4 à 1,5 fois celle de la jambe médiane, de préférence de 0,6 à 1,2, notamment de 0,8 à 1 fois.

La largeur d'une jambe externe sera par exemple de 6 à 12, de préférence de 5 à 10, notamment de 4 à 8, tout particulièrement de 3 à 6 mm pour une jambe externe de forme rectangulaire. Elle sera plus faible pour une jambe externe ayant en section transversale à sa longueur une forme environ triangulaire ou semi-circulaire. Une jambe externe peut être trapézoïdale, c'est-à-dire que sa largeur peut être différente du côté du pont et du côté de son extrémité. Elle sera avantageusement plus large du côté du pont que du côté de son extrémité. Ainsi les valeurs ci-dessus sont celles de la largeur au départ du pont, puisqu'ensuite elle peut diminuer).

Une agrafe préférée ci-dessus aura une jambe externe d'une longueur de 5 à 12 mm et une largeur de 4 à 8 mm.

Lorsque l'agrafe est réalisée en titane ou inox, l'épaisseur d'une jambe externe rectangulaire sera par exemple de 0,5 à 5, de préférence de 0,6 à 4, notamment de 0,7 à 3, tout particulièrement de 1 à 2 mm au niveau du pont pour s'amenuiser à son extrémité libre respectivement à par exemple de 0 à 2, de préférence de 0,1 à 1, notamment de 0,1 à 0,7, tout particulièrement de 0,2 à 0,3 mm. L'épaisseur d'une jambe externe pourra être plus importante dans le cas d'autres formes comme les formes cylindrique ou conique évoquées ci-dessus.

Une agrafe de l'invention peut être ajourée mais de préférence est pleine.

Elle peut être réalisée dans tout type de matériau implantable (titane, inox, polymère, polyétheréthercétone (PEEK), etc. Les différentes surfaces peuvent êtres avantageusement revêtues de titane poreux et/ou d'hydroxyapatite pour une bonne accroche osseuse.

Les jambes externes des agrafes objet de la présente invention ont leur extrémité libre pointue. Selon la forme de l'extrémité libre, il s'agira d'une pointe ou d'une lame en biseau notamment.

Une agrafe de l'invention peut être réalisée en une seule pièce, ce qui la rend simple et peu onéreuse.

A noter que dans la présente demande, classiquement l'article indéfini "un" doit être considéré comme un pluriel générique (signification de "au moins un" ou encore "un ou plusieurs"), sauf lorsque le contexte montre le contraire (1 ou "un seul"). Ainsi, par exemple, lorsque l'on dit ci-dessus que la jambe médiane comporte sur chacune de ses deux faces une série d'aspérités anti-retour, il s'agit de la présence d'une ou plusieurs séries d'aspérités anti-retour.

Les conditions préférentielles de mise en oeuvre de l'invention sont cumulatives, évidemment sauf incohérence que l'homme de l'art peut détecter sans difficulté. En particulier, dans un but de concision, on n'a pas énuméré toutes les différentes combinaisons possibles des dimensions du pont, des jambes et des orifices. Chacune de ces différentes combinaisons doit cependant être considérée comme décrite par la présente description.

Les agrafes objet de la présente invention possèdent de très intéressantes qualités. Notamment leurs jambes externes ayant une extrémité pointue procurent une excellente pénétration dans l'os.

Grâce à leur conception simple, la forme des agrafes et leur taille peuvent être facilement adaptées aux besoins anatomiques de la zone à traiter.

Lorsque l'angle formé par la surface active d'une jambe externe et par la face active adjacente de la jambe médiane va en s'évasant depuis le pont vers les extrémités libres, lors de l'installation pour bloquer des facettes articulaires, la jambe externe de l'agrafe serre contre la jambe médiane l'os dans lequel elle est plantée au fur et à mesure de l'enfoncement dans l'os. Cet angle sera par exemple de 1 à 20, de préférence de 2 à 15, notamment de 3 à 10, tout particulièrement de 4 à 6 degrés.

Une agrafe de l'invention peut glisser le long de l'articulaire sans se planter mais cependant tenir en place grâce aux aspérités anti-retour dont elle est munie.

Les dimensions d'une agrafe ci-dessus (quelques millimètres) permettent l'utilisation de la technique mini-invasive ; en effet l'agrafe peut passer dans un tube de petites dimensions, de 1 à 3 cm de diamètre environ.

Ces qualités sont illustrées ci-après dans la partie expérimentale. Elles justifient l'utilisation des agrafes ci-dessus décrites, dans l'arthrodèse, notamment pour bloquer les facettes articulaires.

C'est pourquoi la présente demande a aussi pour objet un procédé de blocage de facettes articulaires caractérisé en ce que l'on prévoit une agrafe ci-dessus, on insère la jambe médiane dans l'espace inter articulaire de deux vertèbres adjacentes et on plante l'extrémité pointue des jambes externes dans les massifs articulaires des dites vertèbres adjacentes.

Les conditions préférentielles de mise en oeuvre des agrafes ci-dessus décrites s'appliquent également aux autres objets de l'invention visés ci-dessus, notamment au procédé de blocage de facettes articulaires.

L'invention sera mieux comprise si l'on se réfère aux dessins annexés sur lesquels
- la figure 1 représente une agrafe selon l'invention implantée dans l'espace articulaire d'une colonne vertébrale,
- la figure 2 représente une vue en perspective d'une forme de réalisation d'une agrafe selon l'invention,
- la figure 3 représente une vue de côté ou en coupe d'une agrafe de la figure 2,
- les figures 4 et 5 représentent des vues en perspective d'encore une autre forme de réalisation d'une agrafe selon l'invention,
- la figure 6 représente une vue en perspective d'une forme de réalisation d'une agrafe selon l'invention,
- la figure 7 représente une vue de côté ou en coupe d'une agrafe de la figure 2, mettant en évidence un angle formé entre surfaces actives,
- la figure 8 représente une vue en perspective d'une forme de réalisation d'une agrafe selon l'invention.

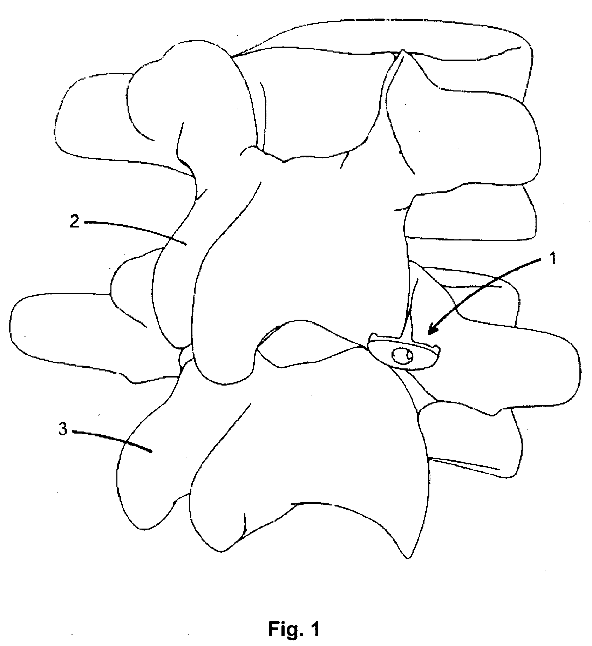

Sur la figure 1, on distingue une agrafe 1 selon l'invention implantée dans l'espace articulaire entre deux vertèbres adjacentes 2, 3 d'une colonne vertébrale.

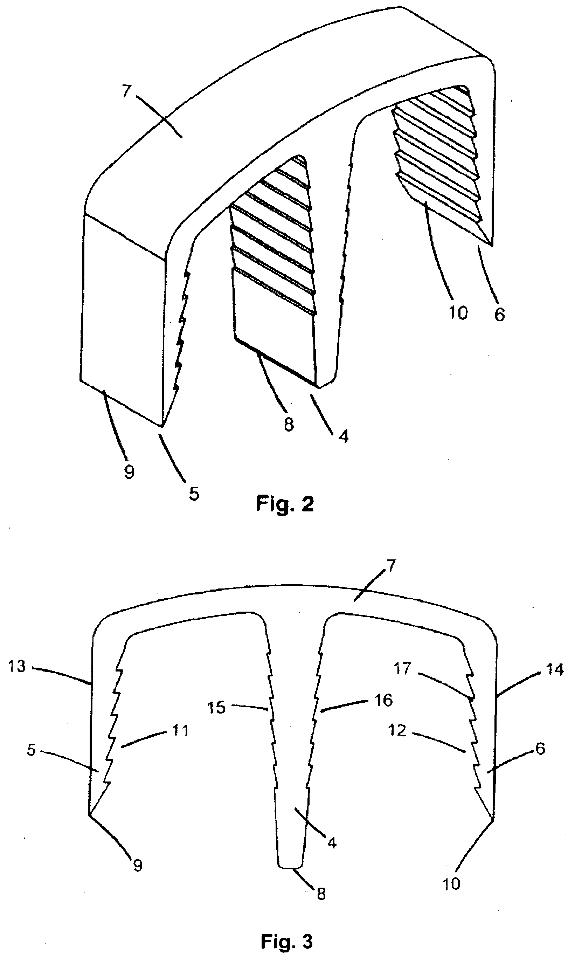

Sur la figure 2, on observe que l'agrafe 1 représentée comprend trois jambes 4, 5, 6 alignées, solidaires d'un pont 7. L'agrafe 1 comprend une jambe médiane 4 et des jambes externes 5, 6. Les jambes 4, 5, 6 ont chacune deux faces et une extrémité libre 8, 9, 10. Les jambes externes 5, 6 ont leur extrémité libre 9, 10, telle que vue en coupe longitudinale ou en élévation comme montré sur la figure 3, qui est pointue. Compte tenu de leur forme de plaques, les jambes ont une extrémité en forme de lame coupante à la manière d'un biseau. Le pont 7 a une forme rectangulaire et est légèrement arqué comme on l'observe mieux sur la figure 3, l'angle formé par les tangentes aux extrémités du pont étant d'environ 35°. Les trois jambes 4, 5, 6, particulièrement la jambe médiane 4, ont une forme de coin. L'agrafe est monobloc, c'est-à-dire réalisée en une seule pièce.

Comme on l'observe mieux sur la figure 3, les jambes externes 5, 6 ont une face interne 11, 12 et une face externe 13, 14, la jambe médiane 4 comportant sur chacune de ses deux faces 15, 16, des aspérités anti-retour qui sont des séries d'encoches 17 en dents de scie formant un système anti-retour. Les jambes externes 5, 6 comportent aussi sur leur face interne 11, 12 une série d'aspérités anti-retour. Comme on le verra ci-après, les sommets des aspérités constituant des surfaces actives, les surfaces actives étant parallèles ou environ parallèles. Les trois jambes 4, 5, 6, particulièrement la jambe médiane 4, ont une forme de coin.

L'épaisseur de la jambe médiane 4 est relativement importante au niveau du pont 7 pour s'amenuiser à son extrémité libre 8 qui n'est pas agressive. Il en est de même pour les jambes externes 5, 6, mais leurs extrémités libres 9, 10 sont agressives car elles se terminent en biseau pour perforer un os et y pénétrer.

En section transversale (donc relativement à leur longueur), les trois jambes ont une forme rectangulaire.

Les dimensions d'une agrafe de la figure 2 ou 3 sont environ les suivantes:
- pont: Longueur 10 mm, largeur 6 mm, épaisseur 2 mm,
- jambes externes: Longueur 8 mm, largeur 6 mm, épaisseur 2 mm au niveau du pont, 0,7 mm au niveau de l'extrémité libre, avant le biseau,
- jambe médiane: Longueur 9 mm, largeur 6 mm, épaisseur 3 mm au niveau du pont, 1 mm au niveau de l'extrémité libre.

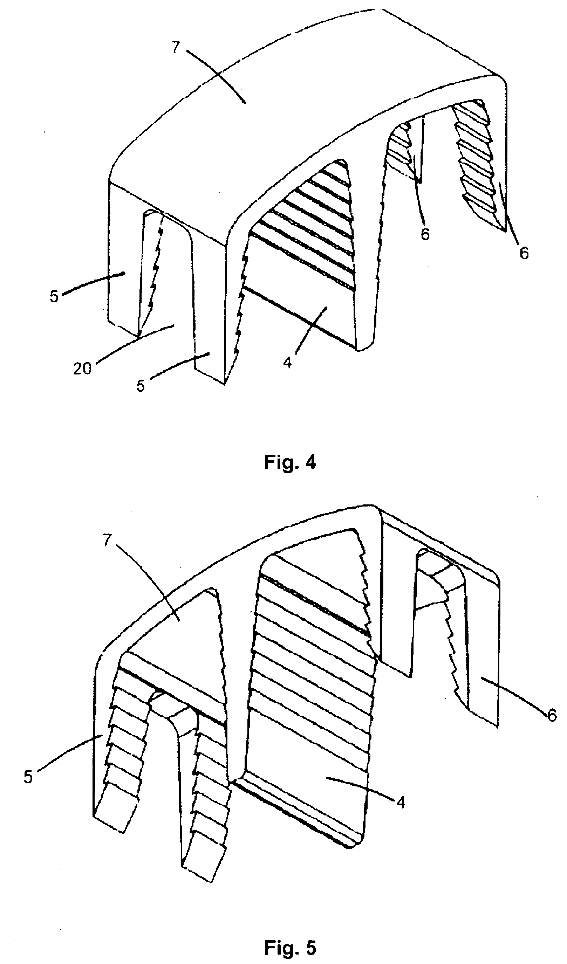

Les figures 4 et 5 représentent des vues en perspective d'encore une autre forme de réalisation d'une agrafe selon l'invention. Dans cette forme de réalisation, les jambes externes 5, 6 sont munies chacune d'un évidement 20 parallèle à la longueur desdites jambe pour produire une agrafe à deux paires de pointes. En d'autres termes, chacune des deux jambes externes est réalisée en deux éléments, prenant ainsi en vue latérale dans la direction de la longueur de l'agrafe une forme de U.

A largeur égale de jambe externe 5, 6, on obtient un aussi bon maintien, et la pénétration dans l'os est plus facile qu'avec une agrafe de la figure 2 ou 3.

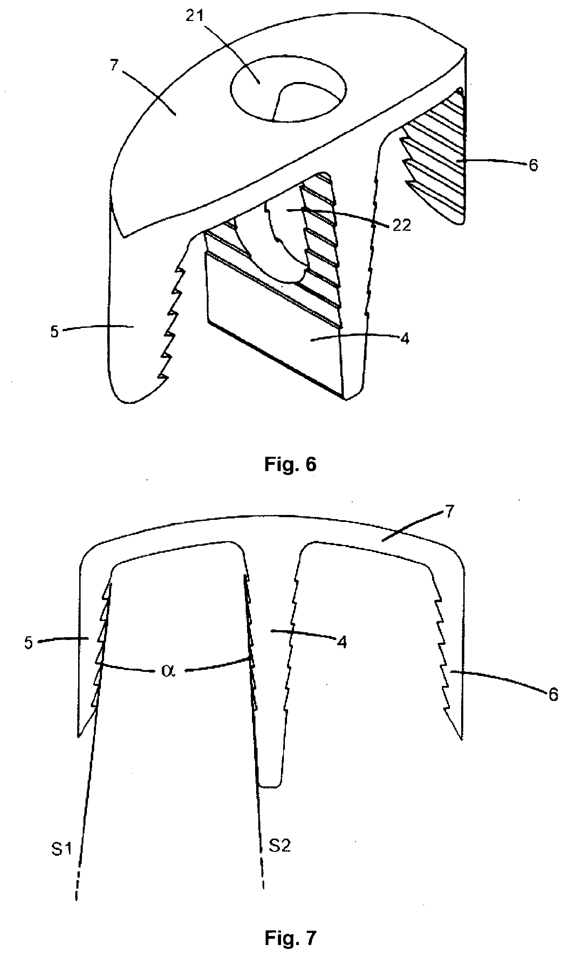

La figure 6 représente une vue en perspective d'encore une autre forme de réalisation d'une agrafe selon l'invention. Les jambes externes 5, 6 ont ici des formes différentes de celles vues précédemment. En section transversale, elles ont une forme environ triangulaire. Elles sont aussi plus épaisses.

Il convient aussi de noter que la jambe médiane 4 et également le pont sont perforés, et les perforations 21, 22 sont en communication. Il est ainsi notamment possible de placer de la greffe osseuse en l'introduisant dans ces perforations 21, 22 en vue d'accélérer l'arthrodèse. Si la perforation 22 est prolongée vers le bas, on obtient une jambe médiane en deux éléments.

Enfin, la surface du pont 7 a une forme ovale et non plus rectangulaire.

La figure 7 analogue à la figure 3 représente une vue de côté ou en coupe d'une agrafe de la figure 2, mettant en évidence l'angle α formé entre surfaces actives S1 et S2. Celles-ci sont les surfaces (ici des plans) définies par les sommets des aspérités. Cet angle α est ici d'environ 10°, montrant que les surfaces actives S1 et S2 sont environ parallèles. L'angle formé par la surface active S1 de la jambe externe 5 et par la face active S2 adjacente de la jambe médiane 4 va en s'évasant depuis le pont vers les extrémités libres. En d'autres termes, le sommet de l'angle formé par ces surfaces actives S1 et S2 est situé du côté du pont 7.

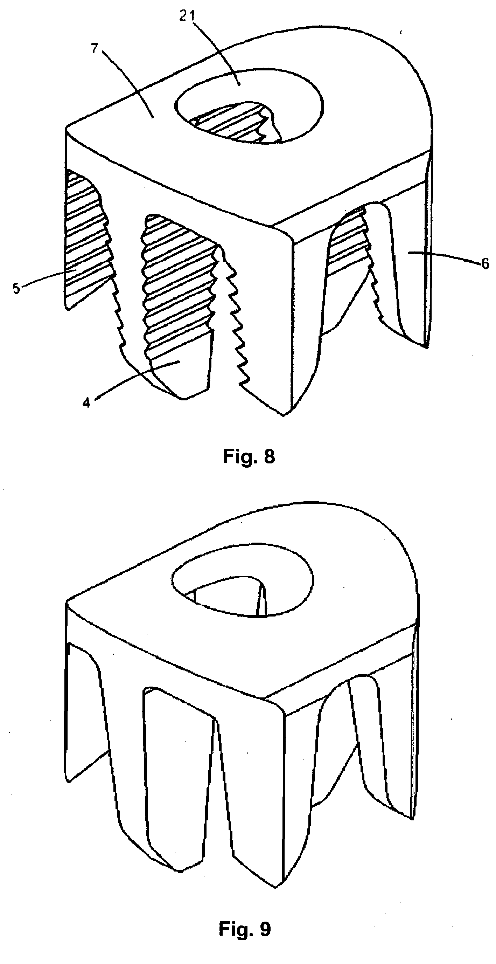

Sur la figure 8, la jambe médiane 4 est réalisée en deux éléments, comme les jambes externes 5, 6 des figures 4 et 5. Dans cette forme de réalisation, la jambe médiane 4 en plusieurs (deux) éléments permet à celle-ci de mieux s'adapter à des formes de surfaces articulaires peu régulières ou anormales. La perforation 21 est en communication avec l'espace vide ainsi créé entre les éléments. Il est ainsi notamment possible de placer de la greffe osseuse en l'introduisant par la perforation 21 en vue d'accélérer l'arthrodèse. La surface de la perforation 21 du pont 7 des figures 7,8 et 9 représente environ 15 % à 30 % de la surface du pont 7. Vue de côté, l'agrafe est comme montrée sur la figure 3, sauf que les jambes externes sont plus épaisses.

Sur la figure 9, la jambe médiane et les jambes externes ne comportent pas d'aspérités anti-retour.

## Revendications

1. Une agrafe de fixation pour facettes articulaires comportant au moins trois jambes (4, 5, 6) alignées solidaires d'un pont (7), dont une jambe médiane (4) et des jambes externes (5, 6), les jambes (4, 5, 6) ayant chacune deux faces et une extrémité libre (8, 9, 10), les jambes externes (5, 6) ayant leur extrémité libre (9, 10) pointue, les jambes externes (5, 6) ayant une face interne et une face externe, la face interne de chacune des jambes externes (5, 6) et les deux faces de la jambe médiane (4) constituant des surfaces actives (S1, S2), les surfaces actives (S1, S2) étant environ parallèles et où la jambe médiane (4) et le pont (7) sont perforés, les perforations (21, 22) étant en communication, pour permettre la greffe de moelle osseuse.

2. Une agrafe selon la revendication 1, **caractérisée en ce qu'**elle est réalisée en une seule pièce.

3. Une agrafe selon la revendication 1 ou 2, **caractérisée en ce que** les jambes externes (5, 6) comportent sur leur face interne une série d'aspérités (17) anti-retour.

4. Une agrafe selon l'une des revendications 1 à 3, **caractérisée en ce que** la jambe médiane (4) comporte sur chacune de ses deux faces une série d'aspérités (17) anti-retour.

5. Une agrafe selon l'une des revendications 1 à 4, **caractérisée en ce que** l'angle α formé par la surface active (S1) d'une jambe externe (5, 6) et par la face active (S2) adjacente de la jambe médiane (4) va en s'évasant depuis le pont (7) vers les extrémités libres (9, 10).

6. Une agrafe selon l'une des revendications 1 à 5, **caractérisée en ce que** chacune des deux jambes externes (5, 6) est réalisée en un seul élément ou en deux éléments.

7. Une agrafe selon l'une des revendications 1 à 5, **caractérisée en ce que** chacune des deux jambes externes (5, 6) est réalisée en deux éléments.

8. Une agrafe selon l'une des revendications 1 à 7, **caractérisée en ce que** la jambe médiane (4) est en forme de coin.

9. Une agrafe selon l'une des revendications 1 à 8, **caractérisée en ce que** la jambe médiane (4) est réalisée en deux éléments.

10. Une agrafe selon l'une des revendications 1 à 9, **caractérisée en ce que** l'angle α formé par la surface active (S1) d'une jambe externe (5, 6) et par la face active (S2) adjacente de la jambe médiane (4) va en s'évasant depuis le pont (7) vers les extrémités libres (9, 10), et a une valeur de 2 à 15 degrés.

11. Une agrafe selon l'une des revendications 1 à 10, **caractérisée en ce que** le pont (7) a une longueur et est arqué dans la direction de sa longueur.

## Patentansprüche

1. Fixierungsklammer für Facettengelenke, umfassend mindestens drei fluchtende Schenkel (4, 5, 6), die fest mit einer Brücke (7) verbunden sind, wobei es sich um einen mittleren Schenkel (4) und äußere Schenkel (5, 6) handelt, wobei die Schenkel (4, 5, 6) jeweils zwei Flächen und ein freies Ende (8, 9, 10) haben, wobei das freie Ende (9, 10) der äußeren Schenkel (5, 6) spitz ist, wobei die äußeren Schenkel (5, 6) eine innere Fläche und eine äußere Fläche haben, wobei die innere Fläche jedes der äußeren Schenkel (5, 6) und die beiden Flächen des mittleren Schenkels (4) aktive Oberflächen (S1, S2) darstellen, wobei die aktiven Oberflächen (S1, S2) ungefähr parallel sind, und wobei der mittlere Schenkel (4) und die Brücke (7) perforiert sind, wobei die Perforationen (21, 22) in Verbindung stehen, um die Knochenmarktransplantation zu gestatten.

2. Klammer nach Anspruch 1, **dadurch gekennzeichnet, dass** sie einteilig ausgeführt ist.

3. Klammer nach Anspruch 1 oder 2, **dadurch gekennzeichnet, dass** die äußeren Schenkel (5, 6) an ihrer inneren Fläche eine Reihe von Rücklaufsperr-Unebenheiten (17) umfassen.

4. Klammer nach einem der Ansprüche 1 bis 3, **dadurch gekennzeichnet, dass** der mittlere Schenkel (4) an jeder seiner beiden Flächen eine Reihe von Rücklaufsperr-Unebenheiten (17) umfasst.

5. Klammer nach einem der Ansprüche 1 bis 4, **dadurch gekennzeichnet, dass** sich der durch die aktive Oberfläche (S1) eines äußeren Schenkels (5, 6) und durch die benachbarte aktive Fläche (S2) des mittleren Schenkels (4) gebildete Winkel α von der Brücke (7) zu den freien Enden (9, 10) geht und sich dabei aufweitet.

6. Klammer nach einem der Ansprüche 1 bis 5, **dadurch gekennzeichnet, dass** jeder der beiden äußeren Schenkel (5, 6) aus einem einzigen Element oder aus zwei Elementen ausgeführt ist.

7. Klammer nach einem der Ansprüche 1 bis 5, **dadurch gekennzeichnet, dass** jeder der beiden äußeren Schenkel (5, 6) aus zwei Elementen ausgeführt ist.

8. Klammer nach einem der Ansprüche 1 bis 7, **dadurch gekennzeichnet, dass** der mittlere Schenkel (4) keilförmig ist.

9. Klammer nach einem der Ansprüche 1 bis 8, **dadurch gekennzeichnet, dass** der mittlere Schenkel (4) aus zwei Elementen ausgeführt ist.

10. Klammer nach einem der Ansprüche 1 bis 9, **dadurch gekennzeichnet, dass** der durch die aktive Oberfläche (S1) eines äußeren Schenkels (5, 6) und durch die benachbarte aktive Fläche (S2) des mittleren Schenkels (4) gebildete Winkel α von der Brücke (7) zu den freien Enden (9, 10) geht und sich dabei aufweitet und einen Wert von 2 bis 15 Grad hat.

11. Klammer nach einem der Ansprüche 1 bis 10, **dadurch gekennzeichnet, dass** die Brücke (7) eine Länge hat und in der Richtung ihrer Länge gebogen ist.

## Claims

1. A fixation clip for articular facets, comprising three aligned legs (4, 5, 6) connected to a bridge (7), namely one central leg (4) and outer legs (5, 6), the legs (4, 5, 6) each having two faces and a free end (8, 9, 10), the outer legs (5, 6) having their free end (9, 10) pointed, the outer legs (5, 6) having an inner face and an outer face, the inner face of each of the outer legs (5, 6) and the two faces of the central leg (4) constituting active surfaces (S1, S2), the active surfaces (S1, S2) being substantially parallel and wherein the central leg (4) and the bridge (7) are perforated, the perforations (21, 22) being in communication in order to make it possible to graft bone marrow.

2. The clip as claimed in claim 1, **characterized in that** it is produced in just one piece.

3. The clip as claimed in claim 1 or 2, **characterized in that** the outer legs (5, 6) have a series of nonreturn asperities (17) on their inner face.

4. The clip as claimed in one of claims 1 to 3, **characterized in that** the central leg (4) has a series of nonreturn asperities (17) on each of its two faces.

5. The clip as claimed in one of claims 1 to 4, **characterized in that** the angle α formed by the active surface (S1) of an outer leg (5, 6) and by the adjacent active face (S2) of the central leg (4) widens from the bridge (7) toward the free ends (9, 10).

6. The clip as claimed in one of claims 1 to 5, **characterized in that** each of the two outer legs (5, 6) is made of a single element or of two elements.

7. The clip as claimed in one of claims 1 to 5, **characterized in that** each of the two outer legs (5, 6) is made of two elements.

8. The clip as claimed in one of claims 1 to 7, **characterized in that** the central leg (4) is wedge-shaped.

9. The clip as claimed in one of claims 1 to 8, **characterized in that** the central leg (4) is produced as two elements.

10. The clip as claimed in one of claims 1 to 9, **characterized in that** the angle α formed by the active surface (S1) of an outer leg (5, 6) and by the adjacent active face (S2) of the central leg (4) widens from the bridge (7) toward the free ends (9, 10), and has a value of from 2 to 15 degrees.

11. The clip as claimed in one of claims 1 to 10, **characterized in that** the bridge (7) has a length and is arched in the direction of its length.
